# EUROPEAN PATENT APPLICATION

(11) **EP 0 739 641 A1**
(43) Date of publication of application: **30.10.1996**
(21) Application number: 96106631.3
(22) Date of filing: 26.04.1996
(51) Int. Cl.: A61M 25/01

(54) **Formable tip guidewire**

(30) Priority: 26.04.1995 US 429533
(71) Applicant: Cordis Corporation, Miami Lakes Florida 33014 (US)
(72) Inventor: Arenas, Alvaro, deceased (US)
(74) Representative: KUHNEN, WACKER & PARTNER

(57) **Abstract**

The formable tip guidewire has a proximal area and a distal area and comprises: a coiled spring wire situated in the distal area; a central core wire having one diameter to which a proximal end of the coiled spring wire is attached and a reduced diameter shaft or rod portion in a distal area of the guidewire; a rounded tip member; a distal end of the rod portion being fixed to the rounded tip member; the coils of the coiled spring wire in the distal area of the guidewire being spaced apart to provide greater flexibility; and, a sleeve member of formable material having a desired degree of plasticity being situated in the distal area of the coiled spring wire within the coils of the coiled spring wire in the distal area and around and in contact with the distal rod portion of the core wire.

## Description

### BACKGROUND OF THE INVENTION

### 1. Field of the Invention.

The present invention relates to a formable tip guidewire having a sleeve of flexible material positioned between a central core wire and an outer coiled spring wire of the guidewire to enable a use of the guidewire to shape the tip portion of the guidewire to a desired shape.

### 2. Description of the related art including information disclosed under 37 CFR §§ 1.97-1.99.

Heretofore, various flexible guidewires have been proposed. Examples of several previously proposed flexible guidewires are disclosed in the following U.S. Patents:

| U.S.Patent No. | Patentee |
|---|---|
| 4,538,622 | Samson et al. |
| 4,545,390 | Leary |
| 4,619,274 | Morrison |
| 4,721,117 | Mar et al. |
| 4,748,986 | Morrison et al. |
| 4,763,647 | Gambale |
| 4,846,186 | Box et al. |
| 5,069,226 | Yamauchi et al. |

The Sampson U.S. Patent No. 4,538,622 discloses a guidewire comprising an elongate flexible cylindrical element, a first coil formed of metallic material and secured to a distal portion of the cylindrical element and a second coil formed of a material which is different from the material of which the first coil is formed and which has higher flexibility than the first coil connected to the distal end of the first coil.

The Leary U.S. Patent No. 4,545,390 discloses a steerable guidewire for a balloon dilatation procedure. The guidewire includes a small diameter flexible rod which is tapered in the distal region thereof. The tapered distal region is surrounded by a helically wound spring which is brazed at its proximal and distal ends to the base and tip of the rod. The distal region of the guidewire can be bent manually by a surgeon into a curved shape which it will tend to assume when relaxed. The distal region of the helically wound spring extends beyond the distal end of the rod and is highly flexible.

The Morrison U.S. Patent No. 4,619,274 discloses a guidewire having a progressively attenuated diameter. The coiled wire has an outer diameter which decreases toward the distal end of the guidewire. Furthermore, the diameter of the wire from which the coil is made decreases in a direction toward the distal end of the guidewire. As a result a small diameter coil wire is provided at the distal end of the guidewire which is highly flexible.

The Mar et al. U.S. Patent No. 4,721,117 discloses a guidewire. The coil of the guidewire increases in flexibility toward the distal end of the guidewire and is fixed to a center core shaft at its proximal and distal ends and at an intermediate point near the distal end.

The Morrison et al. U.S. Patent No. 4,748,986 teaches a floppy guidewire with an opaque tip. Coils at the distal end of a coiled spring wire of the guidewire are spaced or stretched to provide additional flexibility in the distal extremity of the coiled wire.

The Gambale U.S. Patent No. 4,763,647 teaches a dual coil steerable guidewire. A center coil extends from a tapered tip of a center core wire to a tip member and an outer coil which is brazed to the center core wire near the tapered portion thereof and extends to the tip member.

The Box et al. U.S. Patent No. 4,846,186 teaches a guidewire including a core wire and a flexible spring tip.

Yamauchi et al. U.S. Patent No. 5,069,226 teaches a catheter guidewire with a pseudo elastic shaped memory alloy having a desired plasticity at one end. The guidewire comprises a solid core wire and an outer jacket covering the core wire. The guidewire has a pseudo elasticity for enhancing the steerability of the wire.

### SUMMARY OF THE INVENTION

According to the present invention there is provided a formable tip guidewire having a proximal area and a distal area and comprising: a coiled spring wire situated in the distal area; a central core wire having one diameter to which a proximal end of the coiled spring wire is attached and a reduced diameter shaft or rod portion in a distal area of the guidewire; a rounded tip member; a distal end of the rod portion being fixed to the rounded tip member; the coils of the coiled spring wire in the distal area of the guidewire being spaced apart to provide greater flexibility; and, a sleeve member of formable material having a desired degree of plasticity being situated in the distal area of the coiled spring wire within the coils of the coiled spring wire in the distal area and around and in contact with the distal rod portion of the core wire.

### BRIEF DESCRIPTION OF THE DRAWINGS

FIG. 1 is a longitudinal plan view of a prior art flexible guidewire partly in section and with portions broken away.

FIG. 2 is a longitudinal plan view partly in section and with portions broken away of a flexible guidewire constructed according to the teachings of the present invention.

FIG. 3 is an enlarged sectional view of the distal or tip portion of the guidewire shown in FIG. 2.

FIG. 4 is an enlarged sectional view of the distal or tip portion of the guidewire shown in FIG. 2, similar to the view shown in FIG. 3, but with the guidewire bent in the area where a flexible sleeve is positioned between an outer coiled wire and an inner core wire.

FIG. 5 is a plan view of the flexible guidewire shown in FIG. 2 inserted through a balloon catheter and positioned to enter an area of stenosis in a blood vessel, the blood vessel being cut away to show the area of stenosis.

### DESCRIPTION OF THE PREFERRED EMBODIMENT(S)

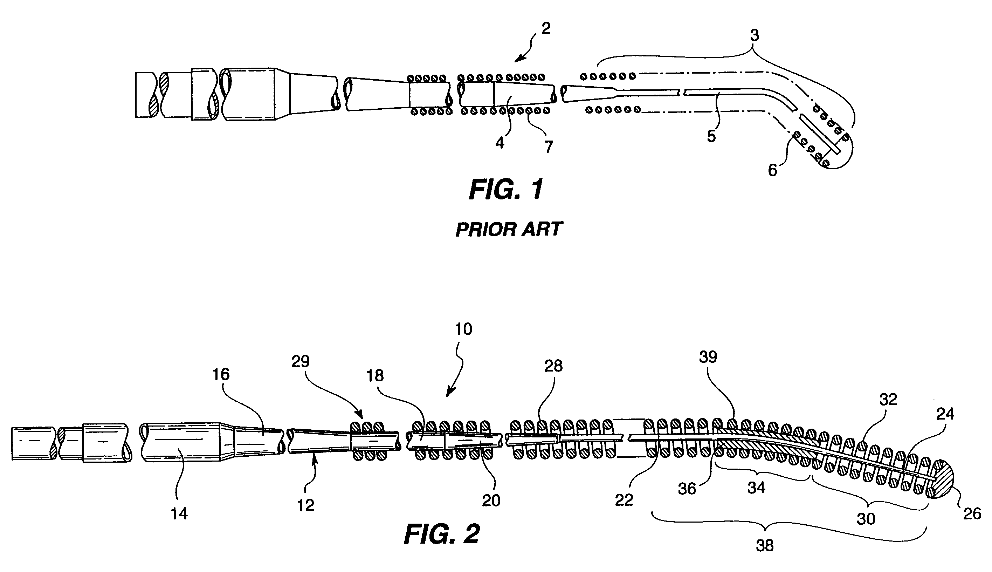

Referring now to the drawings in greater detail FIG. 1 is a longitudinal plan view with portions broken away and partly in section of a prior art guidewire 2 having a flexible spring tip 3. This guidewire 2 can be of the type disclosed in the Box et al. U.S. Patent 4,846,186 or the Palmer U.S. Patent No. 5,174,302. A central core wire 4 may have a flattened shape in a distal end portion 5 of the core wire 4 and coils 6 of a flexible coil spring wire 7 are spaced or stretched in the flexible spring tip 3 to enhance the flexibility of the guidewire 2.

As shown in FIG. 2, a guidewire 10 constructed according to the teachings of the present invention includes a center core wire 12 having a large-in-diameter proximal end portion 14, a tapered portion 16, a smaller-in-diameter cylindrical portion 18 and another tapered portion 20 and an elongate rod or shaft portion 22 which gently tapers to a distal end 24 which is fixed to the back side of a tip member 26. The tip member 26 can be a brazed or soldered weld.

Then, a coiled spring wire 28 is fixed at its proximal end 29 to the smaller-in-diameter center wire portion 18 and extends in a coil to the tip member 26 and fixed thereto.

In a distal end region 30 of the guidewire 10 coils 32 of the coiled spring wire 28 are stretched or spaced to provide increased flexibility. The smaller diameter rod portion 22 of the core wire 12 also enhances the flexibility of the distal end region 30.

A formable/deformable region 34 is provided just proximal to the distal end region 30.

The formable/deformable region 34 is made formable or deformable by the provision of an elongate metal sleeve member 36 which is positioned between the inner diameter of the coils 32 of the coiled spring wire 28 and the center core wire 12 which is preferably straight in that area of the distal rod portion 22 of the core wire 12.

The sleeve member 36 is preferably made of a formable and deformable metal such as a titanium nickel alloy of the type disclosed in the Yamauchi et al U.S. Patent No. 5,069,226. Of course, it will be understood to those skilled in the art that the sleeve member 36 can be made of other metals or even be made of a formable/deformable plastic material.

The sleeve member 36 of flexible material can be located as desired within the coiled spring wire 28 and preferably just behind the distal end region 30. The sleeve member of bendable material preferably has a high plasticity. The sleeve member 36 of flexible material holds the position of the coil segments of the coiled spring wire 28 in the formable/reformable region 34.

This formable/reformable region 34 may be reshaped in any way and the shape will be held.

The extreme distal end region 30 of the coiled spring wire 28 does not contain the flexible sleeve member 34 so that it is more flexible and able to follow the vasculature more easily in the vessel into which the guidewire 10 is inserted.

The flexible sleeve member 34 is shown having a length of approximately 5 mm. However, if desired, it can be longer and extend a substantial length of the guidewire 10 within the coiled spring wire 28 and around the mid portion 22.

A significant advantage of the guidewire 10 constructed according to the teachings of the present invention is that the region 34 just proximal of the distal end region 30 is fully reformable. This allows a physician to choose a desired tip shape he or she requires.

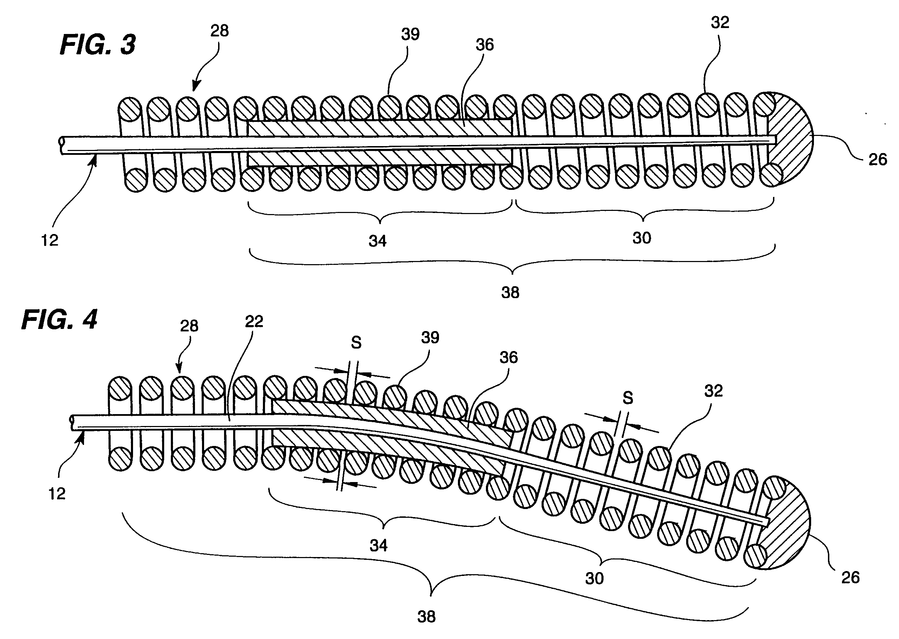

Also the guidewire 10 of the present invention when combined with a forming tool can be formed to very precise shapes and, in fact, the guidewire 10, more specifically a distal area 38 thereof, can be preshaped at the place of manufacture to a desired precise shape. Also it is noted that the spacing between the coils 39 of the coiled spring wire 28 in the formable/reformable region 34 are also spaced or stretched apart a distance S as shown in FIG. 4 to facilitate bending of the guidewire 10 in the formable/reformable region 34.

FIG. 3 shows the distal end portion of the guidewire 10 with the formable/reformable region 34 before it is shaped to a desired configuration.

Then FIG. 4 shows the distal area of the guidewire 10 in the formable/deformable region 34 bent to a desired shape.

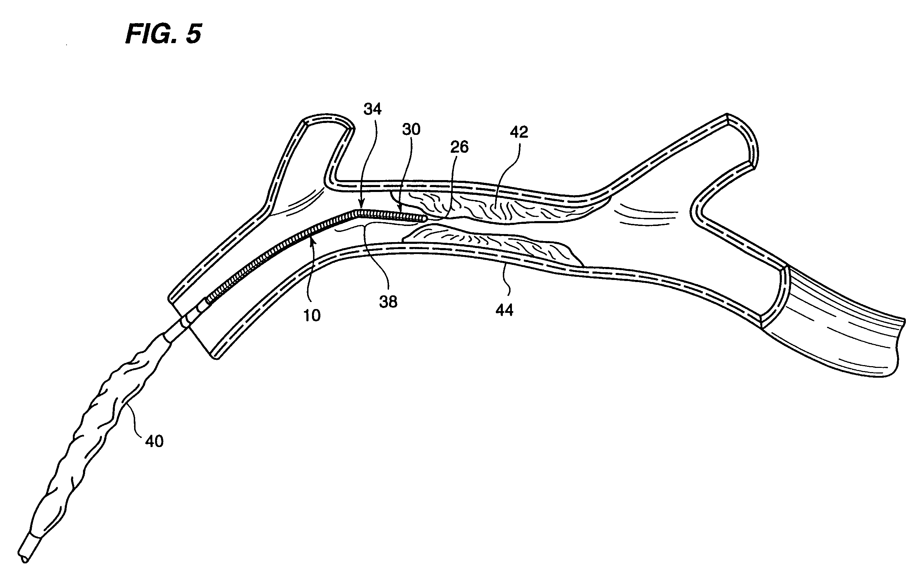

FIG. 5 illustrates the use of the guidewire 10 with a dilatation balloon catheter 40 as it is being inserted into an area of stenosis 42 within a blood vessel 44 in a vascular system with a portion of the blood vessel 44 cut away to better show the guidewire 10 extending from the balloon catheter 44.

From the foregoing description, it will be apparent that the guidewire 10 of the present invention has a number of advantages, some of which have been described above and others of which are inherent in the invention. Also it will be understood that modifications can be made to the guidewire described above without departing from the teachings of the present invention. Accordingly, the scope of the invention is only to be limited as necessitated by the accompanying claims.

## Claims

1. A formable tip guidewire having a proximal area and a distal area and comprising:
a coiled spring wire situated in said distal area;
a central core wire having one diameter to which a proximal end of said coiled spring wire is attached and a reduced diameter shaft or rod portion in a distal area of the guidewire;
a rounded tip member;
a distal end of said rod portion being fixed to said rounded tip member;
the coils of said coiled spring wire in the distal area of the guidewire being spaced apart to provide greater flexibility; and
a member of formable material having a desired degree of plasticity being situated in said distal area of said coiled spring wire within said coils of said coiled spring wire in said distal area and in contact with said distal rod portion of said core wire.

2. The guidewire according to claim 1, wherein said member is a sleeve.

3. The formable tip guidewire according to anyone of claims 1 to 2 wherein a distal end portion of said distal core rod is tapered.

4. The formable tip guidewire according to anyone of claims 2 to 3 wherein said formable sleeve member is made of metal.

5. The formable tip guidewire according to anyone of claims 2 to 4 wherein said formable sleeve member has a length of approximately 5 mm.

6. The formable tip guidewire according to anyone of claims 2 to 5 wherein said formable sleeve member extends a significant length of said distal rod portion of said core wire.

7. The formable tip guidewire according to anyone of claims 2 to 6 wherein said formable sleeve member is made of a titanium nickel alloy.

8. The formable tip guidewire according to anyone of claims 2 to 7 wherein said coils in said distal area are stretched or spaced apart in the formable/deformable region where said sleeve member is situated to enhance bendability in said formable/deformable region.

9. The formable tip guidewire according to anyone of claims 2 to 8 wherein said coiled spring wire has a distal end portion and said formable sleeve member is located just proximal of said distal end portion of said coiled spring member.
